# EUROPEAN PATENT APPLICATION

(11) **EP 4 756 827 A1**
(43) Date of publication of application: **10.06.2026**
(21) Application number: 25221298.0
(22) Date of filing: 05.12.2025
(51) Int. Cl.: G16H 20/40, G16H 40/63, G16H 40/67

(54) **MONITORING AND IDENTIFYING SURGEON CONTROL AND SUGGESTING A TASK THAT MAY BE DONE AUTONOMOUSLY**

(30) Priority: 06.12.2024 US 202418971609
(71) Applicant: Cilag GmbH International, 6300 Zug (CH)
(72) Inventor: SHELTON, IV, Frederick E., Cincinnati, 45242 (US); ADAMS, Shane R., Cincinnati, 45242 (US); COWPERTHWAIT, Matthew David, Cincinnati, 45242 (US); HARRIS, Jason L., Cincinnati, 45242 (US)
(74) Representative: Carpmaels & Ransford LLP

(57) **Abstract**

A surgical system that may be associated with using smart devices for autonomous task performance. The system may include a processor configured to monitor the actions of a surgeon controlling a first smart device during surgery and to identify patterns in the actions. Based on identifying the patterns, the system may determine tasks that are suitable for autonomous performance by a second smart device.

## Description

### CROSS-REFERENCE TO RELATED APPLICATIONS

This application is related to the following, filed contemporaneously, the contents of each of which are incorporated by reference herein:
- Attorney Docket No. END9638USNP1, entitled PROGRESSIVE ADVANCEMENT OF AUTOMATED LEVEL BASED ON LEARNED COMPLIMENTARY ASSISTANCE
- Attorney Docket No. END9638USNP2, entitled ADJUSTING AUTOMATED COOPERATIVE OPERATIONS BASED ON SITUATIONALLY DERIVED CONSTRAINTS,
- Attorney Docket No. END9638USNP3, entitled ASSISTANCE ADVANCEMENT MULTI-SYSTEM INTERACTION,
- Attorney Docket No. END9638USNP5, entitled CONTROL OF INFORMATION FLOW, PRIORITIZATION AND MANIFESTATION OF DATA ASSOCIATED WITH AN ACTIVE HCP INTERACTION SPACE,
- Attorney Docket No. END9638USNP6, entitled ADAPTIVE RETRACTION FORCE CONTROL,
- Attorney Docket No. END9638USNP7, entitled ADJUSTMENT OR DISPLAY OF OPTIONS OF POSITIONAL OR ORIENTATION IMPLICATIONS ON SURGICAL TOOL USAGE, and
- Attorney Docket No. END9638USNPS, entitled ADJUSTMENT OF PHYSIOLOGIC FUNCTION SUPPLEMENTATION CONTROL.

The contents of each of the following are incorporated by reference herein:
- U.S. Patent Application No. 18/810,323 entitled METHOD FOR MULTI-SYSTEM INTERACTION, filed on August 20, 2024;
- U.S. Patent Application No. 18/960,006 entitled METHOD FOR SMART SURGICAL SYSTEMS filed on November 26, 2024; and
- U.S. Patent Application No. 18/954,186 entitled METHOD FOR MULTI-SYSTEM INTERACTION, filed on November 20, 2024.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

A surgical system that may be associated with using smart devices for autonomous task performance. The system may include a processor configured to monitor the actions of a surgeon controlling a first smart device during surgery and to identify patterns in the actions. Based on identifying the patterns, the system may determine tasks that are suitable for autonomous performance by a second smart device.

Upon identifying such tasks, the system may generate control signals to prompt the second smart device to perform the tasks autonomously. This system may receiving task-related data from the first smart device-such as raw data, compiled data, aggregated data, or decision data-and use the data to guide the second smart device's actions. The system may provide notifications to the surgeon, indicating when the second smart device is prepared to perform tasks autonomously, and may await confirmation or allow the surgeon to inhibit the action within a certain time frame.

The system may adapt to variations in the surgical procedure by detecting deviations from identified patterns and prompting the surgeon for guidance on whether to proceed, modify, or suspend the autonomous actions. The second smart device may operate at levels of automation, and the system may switch between the levels based on trigger events to accommodate changing circumstances during surgery.

### BRIEF DESCRIPTION OF THE DRAWINGS

FIG. 1 is a block diagram of a computer-implemented surgical system.
FIG. 2 shows an example surgical system in a surgical operating room.
FIG. 3 illustrates an example surgical hub paired with various systems.
FIG. 4 shows an example situationally aware surgical system.
FIG. 5 illustrates an example surgical system that may include a surgical instrument.
FIG. 6 illustrates a system diagram of a smart surgical system, which may integrate components to support surgical procedures.
FIG. 7 illustrates an example flow diagram of actions conducted by a smart surgical system.
FIG. 8 illustrates an example flow of information and processing within an artificial intelligence/machine learning (AI/ML)-enabled system.

### DETAILED DESCRIPTION

A more detailed understanding may be had from the following description, given by way of example in conjunction with the accompanying drawings.

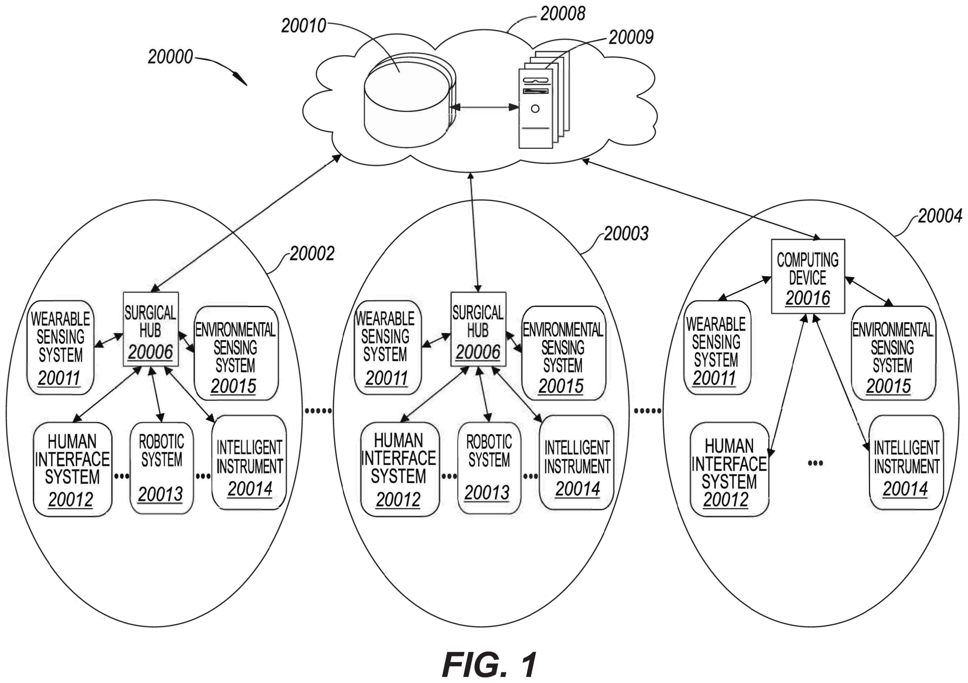

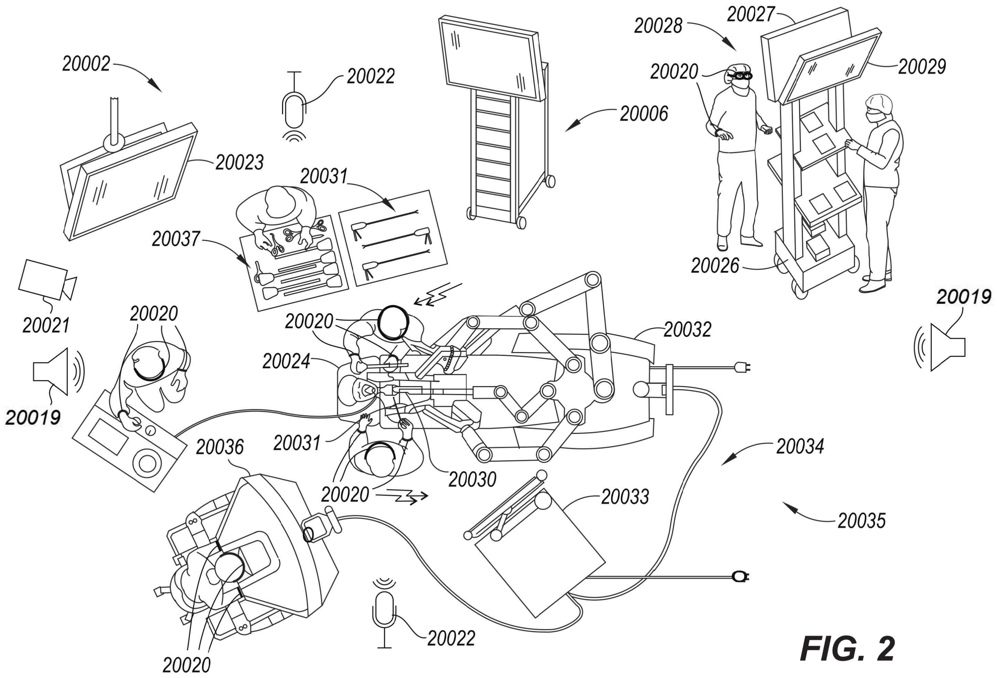

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include, tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, which is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

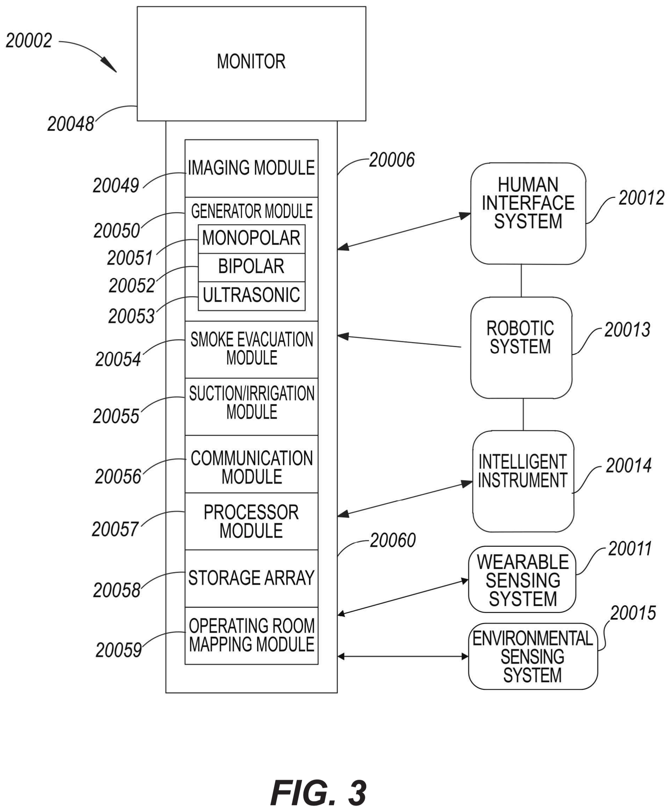

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

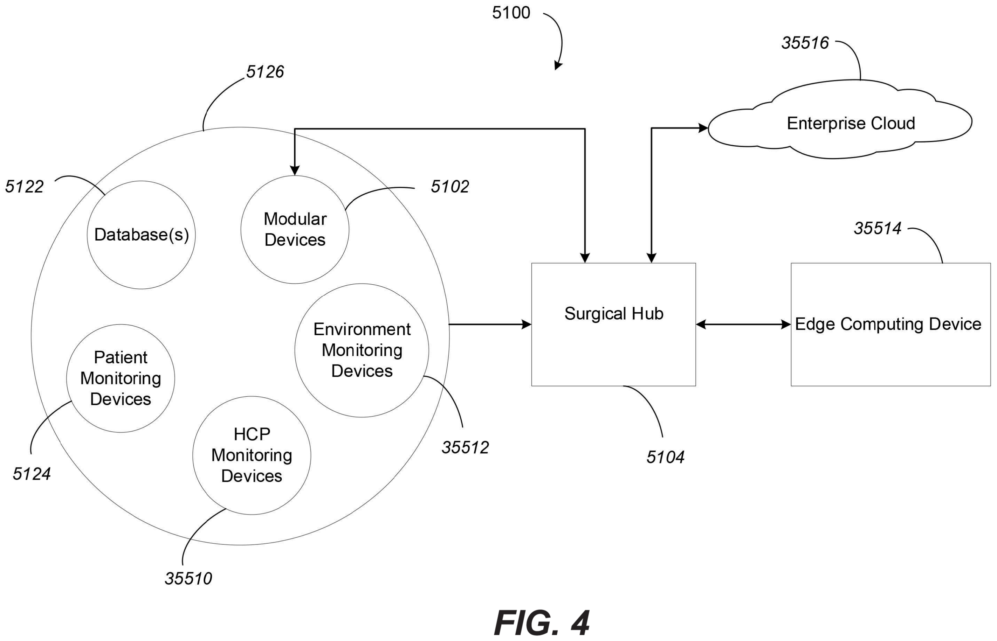

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

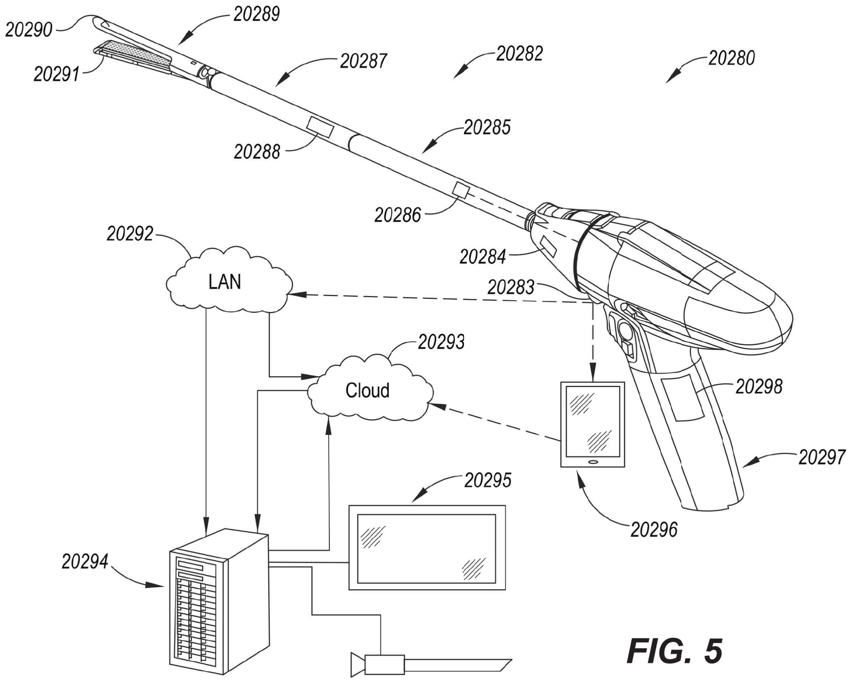

FIG. 5 illustrates an example surgical system 20280 that may include a surgical instrument 20282. The surgical instrument 20282 can be in communication with a console 20294 and/or a portable device 20296 through a local area network 20292 and/or a cloud network 20293 via a wired and/or wireless connection. The console 20294 and the portable device 20296 may be any suitable computing device. Surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

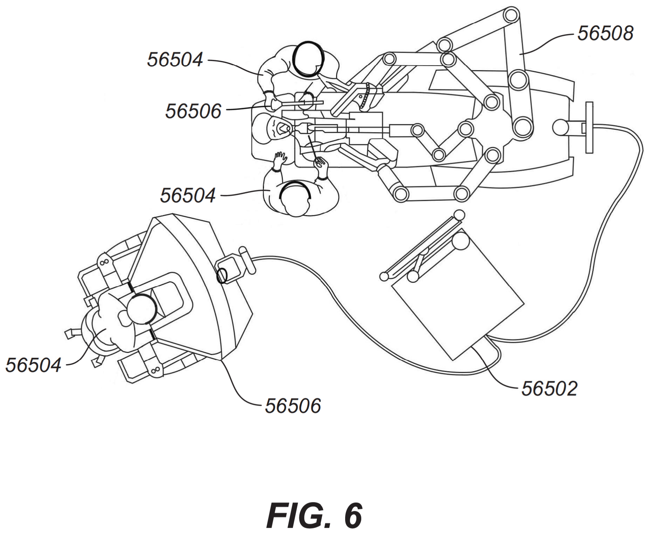

FIG. 6 illustrates a system diagram of a smart surgical system 56502, which may integrate multiple components to support surgical procedures. The system may be connected to a first smart device 56506, and a second smart device 56508, which may interact with a surgical team, represented by surgeons 56504.

The system may monitor the actions, techniques, and reactions of a surgeon during a surgical procedure, focusing on encountered anatomy, physiological patient responses, and surgical steps. The system may identify patterns of control involving multiple smart devices used by the surgeon and may suggest tasks that could be performed autonomously. The interactions between smart devices may include the exchange of data originating from one device and used by another, allowing for synchronization of autonomous actions with actions under surgeon control. The exchanged data may include raw data, compiled data, aggregated data, or decision data, and may be used within a closed-loop control system for the second smart device. A hub or data clearinghouse system may serve as an intermediary between devices, selectively passing on information as needed for control loop operation or adaptation.

The system may monitor healthcare provider utilization, procedural steps, and decisions to detect behavior patterns that allow for the suggestion of minor operations to relieve repetitive tasks, preparations, or setups. The monitoring of user-controlled actions may be associated with suggesting automation based on situational awareness. Learnings may allow the system to adapt across multiple surgeons and accommodating surgeon-specific nuances, such as handedness or technique. Tasks may be considered surgeon agnostic, enabling automation without additional risk. Surgeon-specific learnings may remain isolated to minimize potential conflicts or risks arising from differences in surgical styles.

A database of prior surgical cases may be used to develop a learning model. Existing surgical videos or procedural records may be compiled and reviewed to improve system response. Generalized insights may be shared between systems, and a surgeon's specific preferences and insights gained from a system may be shared across systems used (e.g., other systems used). The procedure plan, the order of tasks or instruments, or surgeon reactions to repeating issues or obstacles may allow for the identification and highlighting of smart device actuations that correspond to a clear sequence or an event trigger. Once recognized, the autonomous system may start and complete the task.

The first smart device 56506, may perform data-capturing functions during a surgical procedure. For example, data from the first smart device may include monitored actions of the surgeons, such as movements or decisions, or physiological responses from a patient. The first smart device may provide task-specific data associated with subsequent processing or task execution.

The second smart device 56508 may serve as an execution device for performing autonomous or semi-autonomous tasks during the surgical procedure. Task execution by the second smart device may be based on control signals generated through an analysis of data collected by the first smart device. For example, the second smart device may execute precision tasks, such as tissue retraction, suturing, or cutting, based on task data or feedback loops.

For example, during a sigmoidectomy, skeletonization or manipulation of the mesentery to free the colon from its fixation points may involve specific sequences including a second smart device. For example, a dual-jaw grasping device may interact with semi-transparent connective tissue to create an opening, enlarge the opening, and clamp tissue for coagulation and transection. A visualization system may monitor tissue interactions, and the dual-jaw device may use tissue impedance to determine whether tissue is inside or outside the jaws. A hub may monitor the procedure plan and communicate key steps to ensure proper sequencing. The energy generator may be triggered automatically to coagulate arteries based on visualization and impedance data, differentiating tissue types and conditions, and refraining from activating for non-arterial tissue.

Tasks suitable for understudy automation may be identified based on factors such as the time associated with surgeon performance, the stress or attention involved, the variability of the task, and the relative risk involved. Actions that are quick or low-risk may be prioritized for automation, and higher-risk tasks may remain under manual surgeon control. For example, understudy automation may be used to finish running a laparoscopic stringing device, throw stitches, or tie knots and may avoid dissecting tissue around critical structures such as the pulmonary artery or vein.

The communication link between the first smart device 56506 and the second smart device 56508 may allow for the exchange of task-related data. Data exchanged between the devices may include raw metrics, aggregated data, compiled analysis, or decision parameters. For example, data regarding tissue location or surgeon intent may be transmitted from the first smart device to the second smart device to inform of task execution.

The surgeons 56504 may retain control of the surgical procedure while interacting with the system. The system may provide the surgeons with notifications or visual indicators to confirm, modify, or halt autonomous actions initiated by the second smart device. For example, notifications may alert the surgeons to variations in task execution or deviations from expected patterns.

When autonomous subsystems with levels (e.g., differing levels) of autonomy interact, the response dynamics within the autonomous network may be influenced by the operational disparity among the systems. Networks with hierarchical levels of autonomy, such as central-peripheral configurations, may align the autonomy of the network to the level of the subsystem with the least advanced capabilities, for example. Such, referred to as autonomy to the lowest common denominator, may allow compatibility. In networks where autonomous responses operate independently, no change may occurs, as a system may retain autonomy and execute tasks without influence from another system.

A subsystem within an autonomous network may adopt a role based on a capability of the subsystem. Full-up autonomy, for example, may represent a autonomous system performing a task without reliance on an external input. An autonomy hypervisor, may emerge from the computing paradigm where a hypervisor manages host system resources, reallocating the host system resources across a virtual machine. In an autonomous system, an autonomy hypervisor may oversee the interaction of subsystem(s), manage hardware allocation, and enable clinically focused applications to operate across hardware platforms.

Horizontal and vertical autonomy may provide a framework for the allocation of software functions within the operating room. Vertical autonomy may align software functions directly with specific hardware for a streamlined operational flow. Horizontal autonomy may allocate software functions to tasks, enabling flexibility and shared hardware utilization. Horizontal and vertical autonomy may coexist or be selectively implemented to benefit operational efficiency based on surgical requirements and the capabilities of the autonomous network.

An autonomy supervisor may play a role in monitoring the routines, error actions, and data flow within the network of subsystems. Basic autonomy supervisors may address the challenges of integrating multiple systems by enabling consistency and reliability in task execution. By supervising the interaction among autonomous subsystems, the network may adapt to varying operational demands while mitigating potential conflicts or errors.

The interaction of multiple smart systems with varying hierarchical levels may be based one which system is in control or holds primary importance for the task at hand. Such hierarchy may shift as the procedure progresses or as patient or surgical risk factors change. The system with the highest priority may assume a leading role, guiding the actions of subsystems or delegating tasks based on the operational context.

Feedback mechanisms may facilitate communication of the current level of automation and upcoming automated actions to healthcare providers in the operating room. The interactions may involve indications of the automation status and defining how surgical staff interacts with autonomous assistance. Communication may include visual indicators, auditory notifications, or graphical displays on user interfaces, providing real-time updates on system performance and readiness. The interactions may allow surgical staff to remain informed and engaged with the autonomous network while maintaining situational awareness during procedures.

Safety protocols within the system may enable autonomous actions by the second smart device to operate within predefined limits. For example, task thresholds or real-time monitoring may minimize unintended outcomes or damage to adjacent structures during task execution. Real-time feedback from the second smart device may inform the surgeons about the progress or status of the task being performed.

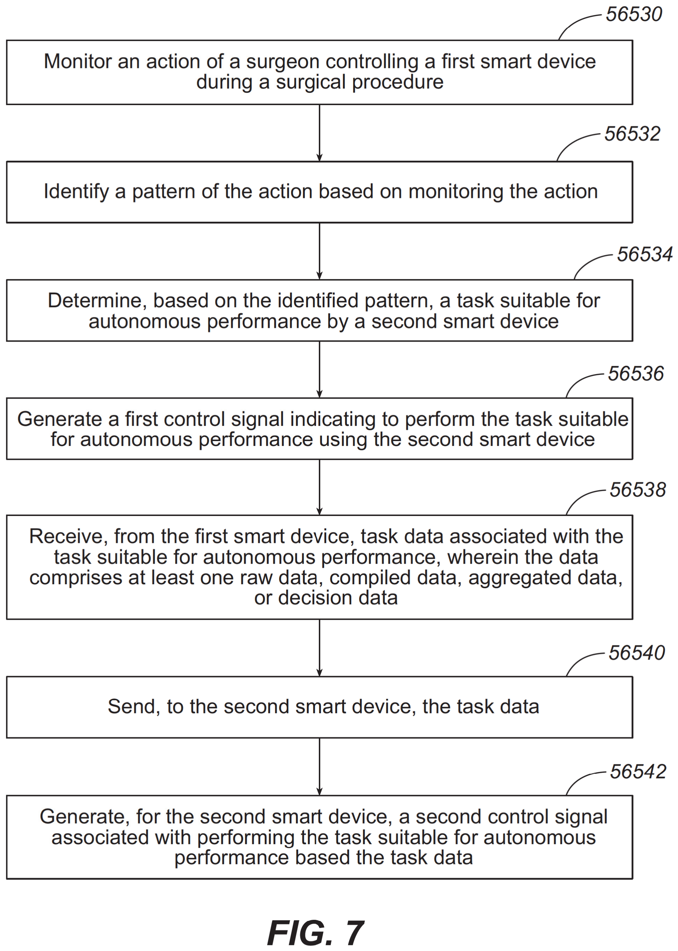

FIG. 7 illustrates an example flow diagram of actions conducted by a smart surgical system. At 56530, the system may monitor an action of a surgeon controlling a first smart device during a surgical procedure. At 56532, the system may identify a pattern of the action based on monitoring the action. At 56534, the system may determine, based on the identified pattern, a task suitable for autonomous performance by a second smart device. At 56536, the system may generate a first control signal indicating to perform the task suitable for autonomous performance using the second smart device. At 56538, the system may receive, from the first smart device, task data associated with the task suitable for autonomous performance, and the data may include at least one raw data, compiled data, aggregated data, or decision data. At 56540, the system may send, to the second smart device, the task data. At 56542, the system may generate, for the second smart device, a second control signal associated with performing the task suitable for autonomous performance based on the task data.

The system may determine a behavior pattern of the surgeon based on the monitored action. The system may determine, based on the determined behavior pattern, an optional modification to the surgical procedure. The system may generate a third control signal indicating the optional modification, and the first control signal may be further generated based on the optional modification.

The system may generate the second control signal based on the task data originating from the first smart device. The system may determine a variation associated with the identified pattern during the surgical procedure. Based on the determination of the variation, the system may generate a third control signal that indicates a confirmation request for whether to proceed with the autonomous performance, modify the autonomous performance, or suspend the autonomous performance. The system may receive a response to the confirmation request and may generate a fourth control signal in accordance with the received response.

The system may generate a third control signal indicating that the second smart device is prepared to perform the task suitable for autonomous performance. The system may monitor for an input indicating inhibition of performing the task suitable for autonomous performance and may determine that the input has not been received within a time threshold. Based on the determination that the input has not been received within the time threshold, the second control signal may be generated.

The system may determine, based on the pattern, that the surgical procedure is being performed out of order. The system may generate a third control signal indicating that the surgical procedure is being performed out of order. The system may determine a surgical task that has been omitted based on the pattern and the determination that the surgical procedure is being performed out of order. The omitted surgical task may include the task suitable for autonomous performance, and the system may generate a fourth control signal indicating the omitted surgical task.

The system may determine that the task suitable for autonomous performance is being performed by the second smart device operating at a first level of automation. The system may detect a trigger event associated with the performance of the task and may generate a third control signal that causes operation of the second smart device to switch from the first level of automation to a second level of automation based on the trigger event.

The system may identify a pattern suitable for automation by evaluating the similarity of an available or operable motion between devices (e.g., a first device and/or a second device). For example, a first smart device performing a repetitive suturing task may exhibit motions that can be replicated by a second device with comparable degrees of freedom and precision. The system may analyze a motion trajectory, a force threshold, and/or a positional accuracy to determine if the task can be transferred to an autonomous or semi-autonomous mode. The evaluation may reduce with a reduction of continuous manual input.

The relevance of the identified pattern to a surgical plan may be used in determining automation suitability. For example, a system may evaluate whether a task aligns with pre-established surgical workflows, such as standardized approaches for tissue dissection or suturing. A task that fits into the surgical plan may be suitable for automation as the task can reduce variability and align with an anticipated outcome. A deviation from the plan may suggest that the task would be suited for human oversight.

The system may assess the degree of fine motor control for a task to determine its suitability for automation. For example, a high-precision task such as threading a needle or manipulating small anatomical structures may include repeated motions that the system can replicate with sub-millimeter accuracy.

The gravity of potential mistakes may influence the determination of automation suitability. Tasks with low risk and high repetition, such as final suturing or securing a wound dressing, may be prioritized for automation. The system may weigh the risk of adverse outcomes when deciding which tasks are appropriate for automation.

The system may incorporate an algorithm that weighs such factors-motion similarity, surgical plan relevance, fine motor control, and risk of mistake-to determine automation suitability. For example, a weighting algorithm may assign higher importance to factors such as low variability and low-risk repetition, while penalizing tasks that require subjective judgment or adaptability to unexpected changes. Such an approach may allow the system to identify candidates for automation in a dynamic surgical environment.

Repetition and consistency of a task may be used in automation determination. For example, a task that involves repeating a single movement, such as stapling or suctioning, may be identified as suitable for automation. The system may recognize a consistent pattern of force application, tool positioning, and timing across a procedure and use such a pattern to suggest or initiate autonomous operation for a task.

The system may analyze a surgeon input to refine the identification of a pattern suitable for automation. For example, a surgeon repeatedly performing a specific sequence of steps may trigger the system to recommend automation for part or all of the sequence. Surgeon feedback on such a recommendation may allow iterative refinement of the system's decision-making process, enabling the system to adapt to an individual surgeon preference or style.

The integration of historical data from previous procedures may assist in identifying an automation candidate. The system may access a database of past cases to recognize patterns across surgeons and procedures, highlighting tasks that are universally repetitive or low risk. For example, the system may analyze (e.g., hundreds of) suturing instances to refine its algorithm for identifying similar tasks in future procedures, so that automation is grounded in empirical data.

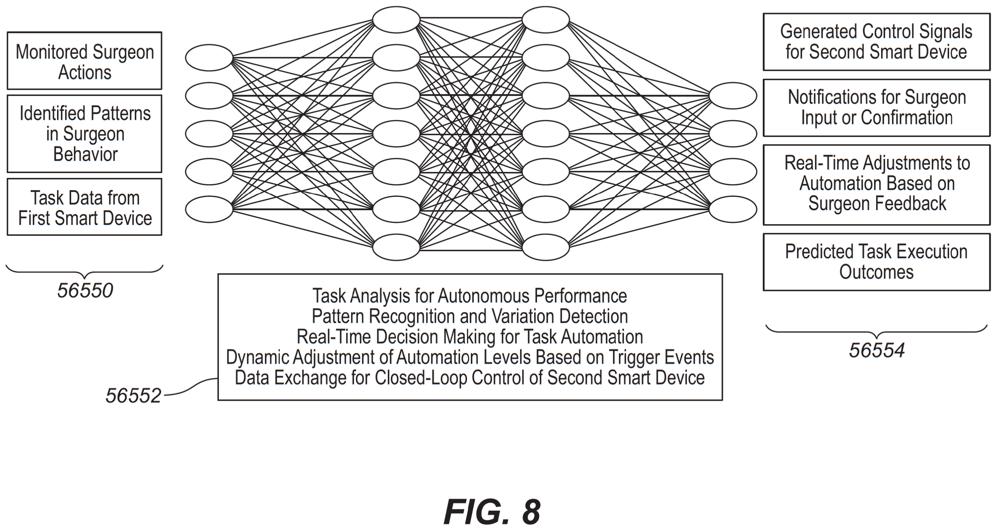

FIG. 8 illustrates an example flow of information and processing within an artificial intelligence/machine learning (AI/ML)-enabled system. FIG. 8 includes the following sections associated with the system: input 56550, processing 56552, and output 56554. The sections may represent how data is acquired, analyzed, and utilized to assist with surgical operations.

Input 56550 may include data sources feeding into the system during a surgical procedure. Data sources in input 56550 may include monitored surgeon actions, identified patterns in surgeon behavior, and task data originating from the first smart device. For example, input 56550 may collect data reflecting a sequence of movements made by a surgeon and correlate the movements with surgical tasks or procedural steps.

The system may acquire or collect related or potentially useful information stored in other hospital systems based on user-monitored actions. For example, if a buttress is planned for use with a stapler cartridge and is not included in the procedural plan, the gopher system may retrieve digital instructions for use (IFU) or details on alternative adjunct products. Such may provide back-table nurses or surgeons with additional information on loading, usage, or available alternatives, ensuring procedural flexibility and support.

The awareness of the surgical staff regarding the current level of assistance provided by the surgical system may be facilitated through notification and display mechanisms. Preoperatively, surgical planning software may assist in the placement of surgeons and automated systems within the operating room, aligning the placement with the preferences and capabilities of the surgeons. During the intraoperative phase, a notification regarding the current level of automation assistance may be presented through a transient display on a device.

At the time of device bootup, the smart system may present its operational status to the user. In a surgical robot, such presentation may occur through a graphical user interface. A handheld device may utilize blinking LEDs to convey levels of assistance, with specific sequences indicating readiness or operational states. When connected to a central surgical hub, the system may provide a message confirming active status and a level of functionality, such as understudy-level data aggregation and accumulation.

A device for automating a surgical task may include a processor configured to perform several specific functions. The processor may receive an indication of a surgical task to be carried out using a surgical instrument, where the capabilities of the surgical instrument may correspond to a plurality of levels of automation. The processor may monitor the performance of the surgical task while the surgical instrument operates at a first level of automation selected from the plurality of levels.

Processing 56552 may include advanced algorithms that analyze the input data. Processing functions may include task analysis, pattern recognition, variation detection, and decision-making for autonomous task execution. For example, an algorithm in processing 56552 may identify deviations in the surgeon's actions from a pre-defined standard and adjust automation parameters accordingly. Processing 56552 may also dynamically update control signals based on detected trigger events.

Processing 56552 may include predictive modeling to assess surgical outcomes or risks associated with specific tasks. For example, processing 56552 may analyze the likely impact of an autonomous task and determine whether additional adjustments are needed to mitigate risk. Processing 56552 may include a feedback loop to enable coordination between devices during the procedure.

The system may observe behavior patterns or techniques exhibited during surgical procedures and may suggest modifications based on identified patterns. When the system identifies a repeating event trigger or a recurring sequence of operations, the system may prompt the user with a visual or audible confirmation request, asking if the user would like the system to execute the identified actions automatically in the future. Such prompts may be based on consistent repetition observed in a particular set of events or sequences.

If the system is operating tasks automatically and detects a variation or change in the sequence, the system may prompt the user to confirm whether the automated step is still desired in light of the variation. The system may ask if the modified behavior should be accepted as an approved variation for future operations or if the user prefers to suspend the automation temporarily or permanently based on the identified variation. The prompts may allow for greater adaptability and user control of the automation process.

The system may determine modifications by leveraging existing datasets. By comparing procedural data against class determining or key opinion leader (KOL) benchmarks, the system may identify potential modifications. Surgeon-agnostic learnings may be utilized to create outcomes beneficial for both the patient and the surgeon, and physics-based modeling may refine automation capabilities by evaluating the comparative efficiencies of the smart system and the surgeon. For example, the system may identify speed improvements in tasks such as running a suture line or tying knots by using closed-loop control to position a needle faster than a surgeon. If the surgeon accepts the suggested improvement, the system may promote the task to a higher level of automation, such as assistant or co-pilot automation.

A gopher system may focus on level 1 tasks, involving the retrieval and display of operational documentation, procedural parameters, or planning details without making changes to the motion or function of equipment in the surgical environment. Menial or secondary tasks for healthcare providers may include adjusting power levels across devices. For example, if the user adjusts the power level on an advanced energy generator, the gopher system may proportionally adjust the speed control on a smoke evacuator to account for the likely increase in smoke plume generation. The system may assist with fetching and integrating patient data and previous history into the procedural plan, interpreting the implications of comorbidities, medications, or physiological factors on specific procedural steps.

Autonomous systems operating at higher levels of autonomy may provide more frequent user notifications or user interactions when encountering unanticipated issues. For example, the system may prompt the user to intervene if an automated task does not align with expected results. At lower autonomy levels, responses to unexpected issues may involve minimal user interaction. The system may temporarily adjust its autonomy level to accommodate unanticipated events, such as when the user is unprepared or lacks the time to provide timely instructions. Such may enable continued task performance while allowing for greater adaptability.

The generalized status of the system in the context of automation assistance may be displayed in in a way that visually or dynamically communicates a current capability. For example, when a surgeon commands an arm to move, the system may begin with a green indicator, transitioning to yellow or red during physical motion to signal changes in its operational state. Status indicators may include text, blinking lights, or icons, providing intuitive updates on system readiness for new commands or tasks.

An automation capability in use may be displayed for clarity. Numbering correlated to automation levels, for example, may be shown on a console, with values ranging from 0-4 to indicate the specific level of automation for each arm. Icon-based displays may offer a visual representation of the system's mode, such as distinguishing between gopher-level assistance and co-pilot-level operation. Pre-selected options available to the user may be mapped to the system capabilities, so that valid choices are presented.

Surgeon interaction with a highly automated system during an unexpected issue may involve a range of tools and options. Quick commands, such as preconfigured text-message-like responses, may allow surgeons to request specific actions from the system, such as camera repositioning for visualization. Manual override options may include an emergency stop button to immediately halt all system movements. For example, pressing the emergency stop button may result in the suspension of all arm movements until the issue is resolved. Options for pausing or resetting the system may be available, with a reset involving the reinitialization of algorithms and retaining mechanical or spatial encoder data.

The processor may detect a trigger event that is associated with the performance of the surgical task. Upon detecting the trigger event, the processor may switch the operation of the surgical instrument from the first level of automation to a second level of automation, with the first level of automation and the second level of automation being part of the levels of automation. Such functionality may allow the surgical instrument to adapt to changing conditions (e.g., requirements) during the surgical task.

Output 56554 may include generated control signals for task execution by the second smart device, notifications for surgeon input, and real-time adjustments to automated actions. Notifications may provide surgeons with actionable information to either confirm or modify the proposed task execution. For example, a notification may alert surgeons that a task is ready for autonomous performance or request input regarding a detected variation.

Without explicitly directing the user to approve the next step in an automated sequence, the system may highlight an indicator on the tool or the screen to show that the next step is ready to be activated. Examples of such indicators may include an LED on the actuation lever of a device or a flashing power level indicator on a display screen. If the user does not inhibit the action within a predetermined time window, the system may automatically execute the next step in the sequence. Such may streamline operations while providing opportunities for manual intervention when necessary.

The system may use indications of sequential tasks or steps to notify the user if the steps are being executed out of their typical order. If the normal sequence of steps, such as A-B-C, is being followed as A-C, the system may notify the user through haptic feedback, audible alerts, or visual notifications to indicate that a step may have been skipped. The system may identify and highlight the missing or overlooked step to aid in procedural completeness and accuracy.

Responses to unexpected issues encountered during autonomous surgical procedures may vary based on the autonomy level at which the system operates. The specific response may depend on several factors, including the risk to the patient of potential complications or unintended secondary outcomes, the operational limits of the device performing the task, the historical frequency of successful or failed outcomes in similar scenarios, the magnitude of the unexpected issue, and the relevance of the issue to the primary objective of the device. The factors may influence the system's decision-making and the degree of user involvement during an unexpected event.

Reactions to unexpected events may depend on the specific equipment available within the operating room. For example, systems integrated with advanced communication capabilities may better coordinate their responses compared to systems lacking such features. The availability and functionality of operating room equipment may play a role in determining the system's ability to adapt to and resolve issues.

The system's response may further depend on whether multiple autonomous systems are interacting within the surgical environment. Autonomous systems with communication capabilities may coordinate their actions to address unexpected issues, and systems without communication capabilities may operate independently. For example, a system with communication capabilities may be used alongside a system with different communication capabilities. In examples, the systems may function interactively in the surgical space without forming a fully communicative network.

Communication between autonomous systems may significantly impact responses to unexpected issues. A localized response may involve a single system addressing an issue without input from other systems. Such responses may be used in networks where communication between systems is unavailable. A qualified localized response may involve a system communicating with systems in the network before independently addressing the issue. Such may allow systems to work in a coordinated manner while maintaining localized control.

In examples involving fully unified responses, an autonomous network may respond collectively to an unexpected failure. For example, when a system encounters a surgical scenario beyond its training data, the system may notify (e.g., other) systems in the network. The network may then transition into a maintenance mode to stabilize the environment. Multi-system responses within a network may involve unique or collaborative actions from multiple systems to address unexpected events. The responses may vary based on the nature of the unexpected failure and the communication capabilities of the systems involved.

Consistent status displays may be maintained across the system, providing a real-time update for overall system operations and individual subsystems. Notifications may occur when an arm reverts to manual control, such as transitioning back to gopher mode during manual overrides. Deviation from targeted automation capabilities may be flagged, with alerts like blinking red lights, text indicators, or icons notifying staff of failures to achieve selected assistance levels. Successful selections may be marked with green checkmarks, and discrepancies may use exclamation points.

Postoperative feedback mechanisms may facilitate learning and refinement of the system's automation levels. Manual adjustments performed by the surgeon during the procedure may be documented, and opportunities for surgeon feedback may be presented through surveys or feedback buttons. Reports on automation performance may detail cumulative feedback, highlighting areas of success or improvement (e.g., needed improvement). Such feedback may enable the system's ability to adapt and provide refined assistance in future procedures.

Notifications to surgical staff regarding unexpected issues may be tailored to the type and severity of the issue. Equipment failures, such as hardware failures, software execution errors, or mitigation trips, may prompt notifications. Surgical scenarios not recognized by the system's autonomy or situations where the autonomy level (e.g., required) exceeds the system's capacity may trigger notifications. Notifications may be delivered via a console, monitor, or terminal-based indications, including overlays on the surgeon's monitor, or through text messages or email notifications to staff. Indications may be sent to an IT server, enabling hospital-based notifications. LED or lighting changes on a robotic arm may indicate a transition from a fully autonomous state to a faulted or alert state. Functional shutdowns may be implemented, isolating features until the issue is resolved or disabling non-critical features to focus resources on critical functions.

Acknowledgment or resolution-based mechanisms may be used to manage system disablement. Certain features may remain disabled until the warning or error is acknowledged, while others may use full resolution for reactivation. Haptic-based feedback mechanisms may involve low-level vibrations in handheld devices or surgical console tools to indicate a fault or alert state. Audible notifications may involve tones or escalating alarms to prompt immediate attention from the surgical staff. Escalated notification attempts may increase over time if the issue remains unresolved, progressing from low-priority alerts to more urgent notifications. Notifications may be sent to internal hospital departments such as biomedical engineering or to external parties, including the manufacturer or service representatives, to address the criticality of the issue.

Notifications to the manufacturer, service vendor, or sales representative of the system may include details on the time for acknowledgment and the troubleshooting actions taken by hospital staff. Notifications may document resolution status, creating a record of issue management for reference. The system may utilize a layered approach to notifications, beginning with internal hospital notifications and progressing to external notifications based on the criticality of the issue.

Transitioning between manual and automated control may involve clutch mechanisms or other methods to temporarily suspend automation for manual adjustments. Systems may detect manual inputs from the surgeon console and pause automated functions until the input is completed. Manual and automated operations may be seamlessly integrated, allowing the surgeon to perform adjustments while the system maintains awareness of spatial relationships and task sequencing. Feedback mechanisms may notify the surgeon of changes in the automation state or readiness to resume automated operations.

The system may provide feedback mechanisms for adjustments, such as surgeon-requested actions or manual overrides. For example, a surgeon may command the system to retract a portion of tissue, manually fine-tune the adjustment, and transition control back to the automated system. The system may handle transitions between manual and automated control by detecting when a manual adjustment has been completed and resuming automation in accordance with the surgical plan. Indications of manual adjustments may be recorded, with opportunities for surgeon feedback to refine system behavior.

Real-time adjustments to the automation may be included in output 56734. For example, control signals may update the task being performed based on surgeon feedback or newly detected conditions. Output 56734 may include predicted outcomes of task execution and provide actionable metrics or insights regarding task progression or completion. The systems and methods described herein may be capable of generating a plurality of control signals to serve more than one specific purpose, and references to ordinal-labelled control signals for fulfilling one or more functions need not be read as mutually exclusive. For instance, insofar that the appended claims may include e.g., a first claim referencing "first and second" control signals, a second claim (dependent on the first claim) referencing a "third control signal", and a third claim (dependent on the first or second claim) also referencing its own "third control signal", the latter may be regarded as exactly that when said third claim depends directly on the first claim, but can be regarded effectively as a reference to a "fourth control signal" when the third claim depends on the second claim, unless prohibited by context. It will be appreciated by the skilled person that all present dependent claims may readily be so construed when read in combination with one another.
The following list of numbered Examples forms part of the present disclosure:
1. A surgical system, the surgical system comprising:
   a processor configured to:
   monitor an action of a surgeon controlling a first smart device during a surgical procedure;
   identify a pattern of the action based on monitoring the action;
   determine, based on the identified pattern, a task suitable for autonomous performance by a second smart device;
   generate a first control signal indicating to perform the task suitable for autonomous performance using the second smart device;
   receive, from the first smart device, task data associated with the task suitable for autonomous performance, wherein the data comprises at least one of raw data, compiled data, aggregated data, or decision data;
   send, to the second smart device, the task data; and
   generate, for the second smart device, a second control signal associated with performing the task suitable for autonomous performance based on the task data.
2. The surgical system of Example 1, wherein the processor is further configured to:
   determine a behavior pattern of the surgeon based on the monitored action;
   determine, based on the determined behavior pattern, an optional modification to the surgical procedure; and
   generate a third control signal indicating the optional modification, wherein the first control signal is further generated based on the optional modification.
3. The surgical system of Example 1, wherein the second control signal is generated based on the task data originating from the first smart device.
4. The surgical system of Example 1, wherein the processor is further configured to:
   determine a variation associated with the identified pattern during the surgical procedure;
   based on determining the variation, generate a third control signal indicating a confirmation request for whether to proceed with the autonomous performance, to modify the autonomous performance, or to suspend the autonomous performance;
   receive a response to the confirmation request; and
   generate a fourth control signal in accordance with the received response.
5. The surgical system of Example 1, wherein the processor is further configured to:
   generate a third control signal indicating that the second smart device is prepared to perform the task suitable for autonomous performance;
   monitor for an input indicating inhibition performing the task suitable for autonomous performance; and
   determine that the input has not been received within a time threshold, wherein the second control signal is generated based on the determination that the input has not been received within the time threshold.
6. The surgical system of Example 1, wherein the processor is further configured to:
   determine, based on the pattern, that the surgical procedure is being performed out of order;
   generate a third control signal indicating that the surgical procedure is being performed out of order;
   determine a surgical task that has been omitted based on the pattern and based on the determination that the surgical procedure is being performed out of order, wherein the surgical task that has been omitted comprises the task suitable for autonomous performance; and
   generate a fourth control signal indicating the surgical task that has been omitted.
7. The surgical system of Example 1, wherein the processor is further configured to:
   determine that the task suitable for autonomous performance is being performed by the second smart device operating at a first level of automation;
   detect a trigger event associated with the performance of the task; and
   generate a third control signal that causes operation of the second smart device to switch from the first level of automation to a second level of automation based on the trigger event.
8. A method for a surgical system, the method comprising:
   monitor an action of a surgeon controlling a first smart device during a surgical procedure;
   identify a pattern of the action based on monitoring the action;
   determining, based on the identified pattern, a task suitable for autonomous performance by a second smart device;
   generating a first control signal indicating to perform the task suitable for autonomous performance using the second smart device;
   receiving, from the first smart device, task data associated with the task suitable for autonomous performance, wherein the data comprises at least one of raw data, compiled data, aggregated data, or decision data;
   sending, to the second smart device, the task data; and
   generating, for the second smart device, a second control signal associated with performing the task suitable for autonomous performance based on the task data.
9. The method of Example 8, wherein the method comprises:
   determining a behavior pattern of the surgeon based on the monitored action;
   determining, based on the determined behavior pattern, an optional modification to the surgical procedure; and
   generating a third control signal indicating the optional modification, wherein the first control signal is further generated based on the optional modification.
10. The method of Example 8, wherein the second control signal is generated based on the task data originating from the first smart device.
11. The method of Example 8, wherein the method further comprises:
   determining a variation associated with the identified pattern during the surgical procedure;
   based on determining the variation, generating a third control signal indicating a confirmation request for whether to proceed with the autonomous performance, to modify the autonomous performance, or to suspend the autonomous performance;
   receiving a response to the confirmation request; and
   generating a fourth control signal in accordance with the received response.
12. The method of Example 8, wherein the method comprises:
   generating a third control signal indicating that the second smart device is prepared to perform the task suitable for autonomous performance;
   monitoring for an input indicating inhibition performing the task suitable for autonomous performance; and
   determining that the input has not been received within a time threshold, wherein the second control signal is generated based on the determination that the input has not been received within the time threshold.
13. The method of Example 8, wherein the method comprises:
   determining, based on the pattern, that the surgical procedure is being performed out of order;
   generating a third control signal indicating that the surgical procedure is being performed out of order;
   determining a surgical task that has been omitted based on the pattern and based on the determination that the surgical procedure is being performed out of order, wherein the surgical task that has been omitted comprises the task suitable for autonomous performance; and
   generating a fourth control signal indicating the surgical task that has been omitted.
14. The method of Example 8, wherein the method comprises:
   determining that the task suitable for autonomous performance is being performed by the second smart device operating at a first level of automation;
   detecting a trigger event associated with the performance of the task; and
   generating a third control signal that causes operation of the second smart device to switch from the first level of automation to a second level of automation based on the trigger event.
15. A surgical system, the surgical system comprising:
   a processor configured to:
   identify a pattern of an action of a surgeon controlling a first smart device during a surgical procedure;
   determine, based on the identified pattern, a task suitable for autonomous performance by a second smart device;
   generate a first control signal indicating to perform the task suitable for autonomous performance using the second smart device;
   receive, from the first smart device, task data associated with the task suitable for autonomous performance, wherein the data comprises at least one of raw data, compiled data, aggregated data, or decision data;
   generate, for the second smart device, a second control signal associated with performing the task suitable for autonomous performance based on the task data.
16. The surgical system of Example 1, wherein the processor is further configured to:
   determine a behavior pattern of the surgeon based on the action;
   determine, based on the determined behavior pattern, an optional modification to the surgical procedure; and
   generate a third control signal indicating the optional modification, wherein the first control signal is further generated based on the optional modification.
17. The surgical system of Example 1, wherein the second control signal is generated based on the task data originating from the first smart device.
18. The surgical system of Example 1, wherein the processor is further configured to:
   determine a variation associated with the identified pattern during the surgical procedure;
   based on determining the variation, generate a third control signal indicating a confirmation request for whether to proceed with the autonomous performance, to modify the autonomous performance, or to suspend the autonomous performance;
   receive a response to the confirmation request; and
   generate a fourth control signal in accordance with the received response.
19. The surgical system of Example 1, wherein the processor is further configured to:
   generate a third control signal indicating that the second smart device is prepared to perform the task suitable for autonomous performance;
   monitor for an input indicating inhibition performing the task suitable for autonomous performance; and
   determine that the input has not been received within a time threshold, wherein the second control signal is generated based on the determination that the input has not been received within the time threshold.
20. The surgical system of Example 1, wherein the processor is further configured to:
   determine, based on the pattern, that the surgical procedure is being performed out of order;
   generate a third control signal indicating that the surgical procedure is being performed out of order;
   determine a surgical task that has been omitted based on the pattern and based on the determination that the surgical procedure is being performed out of order, wherein the surgical task that has been omitted comprises the task suitable for autonomous performance; and
   generate a fourth control signal indicating the surgical task that has been omitted.

## Claims

1. A surgical system, the surgical system comprising:
a processor configured to:
monitor an action of a surgeon controlling a first smart device during a surgical procedure;
identify a pattern of the action based on monitoring the action;
determine, based on the identified pattern, a task suitable for autonomous performance by a second smart device;
generate a first control signal indicating to perform the task suitable for autonomous performance using the second smart device;
receive, from the first smart device, task data associated with the task suitable for autonomous performance, wherein the task data comprises at least one of raw data, compiled data, aggregated data, or decision data;
send, to the second smart device, the task data; and
generate, for the second smart device, a second control signal associated with performing the task suitable for autonomous performance based on the task data.

2. A computer-readable medium comprising instructions which, when executed by a computer, cause the computer to:
monitor an action of a surgeon controlling a first smart device during a surgical procedure;
identify a pattern of the action based on monitoring the action;
determine, based on the identified pattern, a task suitable for autonomous performance by a second smart device;
generate a first control signal indicating to perform the task suitable for autonomous performance using the second smart device;
receive, from the first smart device, task data associated with the task suitable for autonomous performance, wherein the task data comprises at least one of raw data, compiled data, aggregated data, or decision data;
send, to the second smart device, the task data; and
generate, for the second smart device, a second control signal associated with performing the task suitable for autonomous performance based on the task data.

3. The surgical system of claim 1 or computer-readable medium of claim 2, wherein the processor is further configured to, or the instructions further cause the computer to:
determine a behavior pattern of the surgeon based on the monitored action;
determine, based on the determined behavior pattern, an optional modification to the surgical procedure; and
generate a third control signal indicating the optional modification, wherein the first control signal is further generated based on the optional modification.

4. The surgical system or computer-readable medium of any one of claims 1 to 3, wherein the second control signal is generated based on the task data originating from the first smart device.

5. The surgical system or computer-readable medium of any one of claims 1 to 4, wherein the processor is further configured to, or the instructions further cause the computer to:
determine a variation associated with the identified pattern during the surgical procedure;
based on determining the variation, generate a third control signal indicating a confirmation request for whether to proceed with the autonomous performance, to modify the autonomous performance, or to suspend the autonomous performance;
receive a response to the confirmation request; and
generate a fourth control signal in accordance with the received response.

6. The surgical system or computer-readable medium of any one of claims 1 to 5, wherein the processor is further configured to, or the instructions further cause the computer to:
generate a third control signal indicating that the second smart device is prepared to perform the task suitable for autonomous performance;
monitor for an input indicating inhibition performing the task suitable for autonomous performance; and
determine that the input has not been received within a time threshold, wherein the second control signal is generated based on the determination that the input has not been received within the time threshold.

7. The surgical system or computer-readable medium of any one of claims 1 to 6, wherein the processor is further configured to, or the instructions further cause the computer to:
determine, based on the pattern, that the surgical procedure is being performed out of order;
generate a third control signal indicating that the surgical procedure is being performed out of order;
determine a surgical task that has been omitted based on the pattern and based on the determination that the surgical procedure is being performed out of order, wherein the surgical task that has been omitted comprises the task suitable for autonomous performance; and
generate a fourth control signal indicating the surgical task that has been omitted.

8. The surgical system or computer-readable medium of any one of claims 1 to 7, wherein the processor is further configured to, or the instructions further cause the computer to:
determine that the task suitable for autonomous performance is being performed by the second smart device operating at a first level of automation;
detect a trigger event associated with the performance of the task; and
generate a third control signal that causes operation of the second smart device to switch from the first level of automation to a second level of automation based on the trigger event.

9. A surgical system, the surgical system comprising:
a processor configured to:
identify a pattern of an action of a surgeon controlling a first smart device during a surgical procedure;
determine, based on the identified pattern, a task suitable for autonomous performance by a second smart device;
generate a first control signal indicating to perform the task suitable for autonomous performance using the second smart device;
receive, from the first smart device, task data associated with the task suitable for autonomous performance, wherein the data comprises at least one of raw data, compiled data, aggregated data, or decision data; and
generate, for the second smart device, a second control signal associated with performing the task suitable for autonomous performance based on the task data.

10. A computer-readable medium comprising instructions which, when executed by a computer, cause the computer to:
identify a pattern of an action of a surgeon controlling a first smart device during a surgical procedure;
determine, based on the identified pattern, a task suitable for autonomous performance by a second smart device;
generate a first control signal indicating to perform the task suitable for autonomous performance using the second smart device;
receive, from the first smart device, task data associated with the task suitable for autonomous performance, wherein the data comprises at least one of raw data, compiled data, aggregated data, or decision data; and
generate, for the second smart device, a second control signal associated with performing the task suitable for autonomous performance based on the task data.

11. The surgical system of claim 9 or computer-readable medium of claim 10, wherein the processor is further configured to, or the instructions further cause the computer to:
determine a behavior pattern of the surgeon based on the action;
determine, based on the determined behavior pattern, an optional modification to the surgical procedure; and
generate a third control signal indicating the optional modification, wherein the first control signal is further generated based on the optional modification.

12. The surgical system or computer-readable medium of any one of claims 9 to 11, wherein the second control signal is generated based on the task data originating from the first smart device.

13. The surgical system or computer-readable medium of any one of claims 9 to 12, wherein the processor is further configured to, or the instructions further cause the computer to:
determine a variation associated with the identified pattern during the surgical procedure;
based on determining the variation, generate a third control signal indicating a confirmation request for whether to proceed with the autonomous performance, to modify the autonomous performance, or to suspend the autonomous performance;
receive a response to the confirmation request; and
generate a fourth control signal in accordance with the received response.

14. The surgical system or computer-readable medium of any one of claims 9 to 13, wherein the processor is further configured to, or the instructions further cause the computer to:
generate a third control signal indicating that the second smart device is prepared to perform the task suitable for autonomous performance;
monitor for an input indicating inhibition performing the task suitable for autonomous performance; and
determine that the input has not been received within a time threshold, wherein the second control signal is generated based on the determination that the input has not been received within the time threshold.

15. The surgical system or computer-readable medium of any one of claims 9 to 14, wherein the processor is further configured to or the instructions further cause the computer to:
determine, based on the pattern, that the surgical procedure is being performed out of order;
generate a third control signal indicating that the surgical procedure is being performed out of order;
determine a surgical task that has been omitted based on the pattern and based on the determination that the surgical procedure is being performed out of order, wherein the surgical task that has been omitted comprises the task suitable for autonomous performance; and
generate a fourth control signal indicating the surgical task that has been omitted.
